# FASCICULE DE BREVET EUROPEEN

(11) **EP 1 879 656 B1**
(45) Date de publication et mention de la délivrance du brevet: **25.04.2012**
(21) Numéro de dépôt: 06744739.1
(22) Date de dépôt: 15.05.2006
(51) Int. Cl.: A61P 31/12, A61P 33/00, A61P 33/10, A61P 35/00, A61K 45/06, A61K 31/045, A61K 31/05

(54) **COMPOSITION PHARMACEUTIQUE COMPRENANT UN ANTITUMORAL ET UN ACTIF CHOISI NOTAMMENT PARMI LE CARVEOL , LE THYMOL ET LE CARVACROL**
PHARMAZEUTISCHE ZUSAMMENSETZUNG MIT EINEM ANTITUMORALEN MITTEL UND EINEM INSBESONDERE AUS CARVEOL, THYMOL UND CARVACROL AUSGEWÄHLTEN WIRKSTOFF
PHARMACEUTICAL COMPOSITION COMPRISING AN ANTITUMORAL AGENT AND AN ACTIVE AGENT SELECTED ESPECIALLY AMONG CARVEOL, THYMOL AND CARVACROL

(30) Priorité: 13.05.2005 WO PCT/IB2005/001314
(43) Date de publication de la demande: 23.01.2008
(73) Titulaire: Advanced Scientific Developements, 20200 Casablanca (MA)
(72) Inventeur: REMMAL, Adnane, Fes 30000 (MA)
(74) Mandataire: Gallois, Valérie
(86) Numéro de dépôt international: PCT/IB2006/001328
(87) Numéro de publication internationale: WO 2006/120564

(56) Documents cités:
- WO-A-96/40192
- WO-A-02/053138
- US-A1- 2003 064 948
- ZEYTINOGLU, H. ET AL: "Inhibition of DNA synthesis by carvacrol in mouse myoblast cells bearing a human N-RAS oncogene" PHYTOMEDICINE , 10(4), 292-299 CODEN: PYTOEY; ISSN: 0944-7113, 2003, XP004957056
- KOPARAL, A. TANSU ET AL: "Effects of Carvacrol on a Human Non-Small Cell Lung Cancer (NSCLC) Cell Line, A549" CYTOTECHNOLOGY , VOLUME DATE 2004, 43(1-3), 149-154 CODEN: CYTOER; ISSN: 0920-9069, 2003, XP008056740
- CROWELL, PAMELA L. ET AL: "Chemoprevention of mammary carcinogenesis by hydroxylated derivatives of d-limonene" CARCINOGENESIS , 13(7), 1261-4 CODEN: CRNGDP; ISSN: 0143-3334, 1992, XP008056749
- BIELACK S S ET AL: "Impact of scheduling on toxicity and clinical efficacy of doxorubicin: what do we know in the mid-nineties?" EUROPEAN JOURNAL OF CANCER (OXFORD, ENGLAND : 1990) SEP 1996, vol. 32A, no. 10, septembre 1996 (1996-09), pages 1652-1660, XP008067928 ISSN: 0959-8049

## Description

L'invention concerne une composition pharmaceutique comprenant deux substances thérapeutiquement actives dont l'une exerce une action de potentialisation sur l'autre, ainsi que l'utilisation de cette composition.

II est connu que l'efficacité des agents thérapeutiques dépend des doses utilisées, ce qui oblige, dans le cas des résistances partielles à augmenter les doses des agents thérapeutiques pour atteindre l'efficacité recherchée. Cette augmentation de la dose conduit à des problèmes d'apparition d'effets secondaires indésirables et de toxicité aiguë ou chronique, pouvant compliquer considérablement l'état des patients traités.

Cette résistance partielle peut devenir une résistance totale. Dans ce cas, l'augmentation des doses n'a plus aucun effet thérapeutique bénéfique, seuls les effets de toxicité sont observés. Le traitement consiste alors à changer l'agent thérapeutique.
Cette cascade peut se répéter et conduire à la situation la plus grave: la résistance totale à de multiples agents thérapeutiques (multi-drug resistance).

Ainsi, en particulier, les malades immunodéprimés, deviennent de plus en plus difficiles à traiter et leur espérance de vie en est réduite d'autant. De plus, leur confort de vie est largement affecté par l'administration à hautes doses d'agents thérapeutiques.

L'invention a pour but de pallier ces problèmes en proposant d'associer au moins deux substances thérapeutiquement actives, dont l'une potentialise l'activité de l'autre, ce qui permet non seulement d'abaisser les doses de chaque substance thérapeutiquement active mais également de traiter les patients atteints de maladies résistantes.

A cet effet, l'invention propose une composition pharmaceutique caractérisée en ce qu'elle comprend:
- au moins une première substance thérapeutiquement active choisie parmi le carvéol, le thymol, le carvacrol, et les mélanges de ceux-ci, et
- au moins une seconde substance thérapeutiquement active qui est un antitumoral, c'est à dire la doxorubicine.

La première substance thérapeutique peut être obtenue par synthèse chimique ou à partir d'une source végétale.

Une composition antitumorale tout particulièrement préférée est une composition dans laquelle ladite première substance thérapeutiquement active est le carvacrol et l'agent antitumoral est la doxorubicine.

Une autre composition antitumorale tout particulièrement préférée est une composition dans laquelle ladite première substance thérapeutiquement active est le thymol et l'agent antitumoral est la doxorubicine.

Encore une autre composition antitumorale tout particulièrement préférée est une composition dans laquelle ladite première substance thérapeutiquement active est le carvéol et l'agent antitumoral est la doxorubicine.

L'invention propose également une trousse (kit) caractérisée en ce qu'elle contient au moins un premier récipient contenant une première substance thérapeutiquement active choisie parmi le carvéol, le thymol, le carvacrol et les mélanges de ceux-ci, et au moins un second récipient contenant une seconde substance thérapeutiquement active qui est un antitumoral, c'est à dire la doxorubicine.

L'invention propose enfin un traitement d'une affection due à une tumeur caractérisée en ce qu'on administre à un patient atteint d'une affection due à une tumeur, de manière simultanée ou séquentielle au moins une première substance thérapeutiquement active choisie parmi le carvéol, le thymol, le carvacrol, et les mélanges de ceux-ci, et au moins une seconde substance thérapeutiquement active qui est un antitumoral, c'est à dire la doxorubicine.

De préférence, on administre de manière simultanée ou séquentielle, à un patient atteint d'une affection due à une tumeur entre 10 et 200 mg/kg de poids du patient/jour de ladite première substance thérapeutiquement active, et entre 2 et 100 mg/kg de poids du patient/jour de ladite seconde substance thérapeutiquement active qui est un antitumoral, c'est à dire la doxorubicine.

Ladite première substance thérapeutiquement active est choisie parmi le carvacrol et le carvéol et ladite seconde substance thérapeutiquement active est la doxorubicine.

L'invention sera mieux comprise et d'autres buts et avantages de celle-ci apparaîtront plus clairement à la lecture de la description explicative qui suit.

La composition pharmaceutique de l'invention comprend en tant que première substance thérapeutiquement active le thymol, le carvacrol, le carvéol, et leurs mélanges éventuels.

La première substance thérapeutiquement active doit être pure.

Ces composés ont des propriétés antitumorales bien connues en eux-mêmes.

Le thymol, le carvacrol, et le carvéol se trouvent en proportions variées dans différents extraits de plantes aromatiques, c'est-à-dire qu'ils peuvent être purifiés à partir de ces plantes. Cependant, ils peuvent être tout simplement obtenus par synthèse chimique.

Or, les inventeurs ont maintenant découvert que ces composés ont un effet de potentialisation sur de nombreuses substances thérapeutiquement actives dont les antitumoraux connus et déjà utilisés en tant que médicaments spécifiques de cette spécialité.

La seconde substance thérapeutiquement active comprise dans la composition pharmaceutique de l'invention est donc un antitumoral, qui est déjà connu en tant que tel et déjà utilisé en tant que médicament spécifique de cette spécialité et dont l'activité est potentialisée.

Parmi les agents alkylants utiles dans la composition pharmaceutique de l'invention, on peut citer la méchlororéthamine, le cyclophosphamide, l'ifosfamide, le melphalan, et le chlorambucil.

On peut également citer les aziridines tels que le thiothépa, la mitomycine C, et l'aziridinylbenzoquinone (AZQ), les sulfonates alkylés tels le busulfan et les nitrosourées telles que la carmustine (BCNU), et la Iomustine (CCNU) et la fotémustine.

Les sels de platine utiles dans l'invention sont le cisplatine, et le carboplatine.

L'anthracycline utile dans la composition pharmaceutique de l'invention est la doxorubicine (ou adriamycine).

La seconde substance thérapeutiquement active comprise dans la composition pharmaceutique de l'invention est donc un agent antitumoral, qui est déjà connu en tant que tel et dont l'activité est potentialisée par la première substance thérapeutiquement active.

La composition pharmaceutique selon l'invention peut être formulée sous une forme adaptée pour une administration simultanée ou séquentielle desdites au moins première et seconde substances thérapeutiquement actives.

La forme galénique de la composition pharmaceutique de l'invention sera adaptée à son utilisation. Par exemple, elle pourra être utilisée sous la forme de solution, de suspension, de cachet ou autres. Les compositions pour administration parentérale sont généralement des solutions ou des suspensions stériles pharmaceutiquement acceptables qui peuvent éventuellement être préparées extemporanément au moment de l'emploi.

Pour la préparation de solutions ou de suspensions non aqueuses, on peut utiliser des huiles végétales naturelles telles que l'huile d'olive, l'huile de sésame ou l'huile de paraffine ou les esters organiques injectables tels que l'oléate d'éthyle. Les solutions stériles aqueuses peuvent être constituées d'une solution des substances thérapeutiquement actives dans l'eau. Les solutions aqueuses conviennent pour l'administration intraveineuse dans la mesure où le pH est convenablement ajusté et où l'isotonicité est réalisée, par exemple par ajout d'une quantité suffisante de chlorure de sodium ou de glucose.

En effet, étant donné la structure chimique de l'antitumoral, et d'autre part, vu la structure chimique du carvéol, du carvacrol, du thymol, on pense, mais sans vouloir être lié par cette théorie, que le carvéol, le carvacrol, le thymol, et leurs mélanges, interagissent avec les antitumoraux, pour former des complexes ayant une structure qui diffuse plus facilement dans les liquides physiologiques de l'organisme et qui diffuse plus facilement dans le cytoplasme des cellules infectées ciblées.

Mais, il a été démontré que lorsque les différents éléments de la composition pharmaceutique de l'invention sont mélangés en présence de détergents tels que le Tween ou le Triton ou de dissolvants tels que l'éthanol et le DMSO (diméthyl sulphoxide), les molécules actives de la première et de la seconde substance thérapeutiquement active s'associent avec les molécules de détergents et de dissolvants et ne forment pas de complexe de potentiatisation.

Or on a découvert que le complexe de potentialisation se forme lorsqu'on utilise une suspension aqueuse d'agar, en tant que moyen de dispersion par viscosité.

Ainsi, la composition pharmaceutique de la présente invention serait de préférence préparée sans détergent et sans solvant. Par exemple, elle sera mise en suspension aqueuse rendue visqueuse par de l'agar à une concentration non gélifiante, par exemple de 1 à 5 grammes d'agar par litre de suspension.

La composition pharmaceutique de l'invention permet de traiter des affections localisées ou généralisées résistantes aux traitements avec des doses plus faibles de chacune desdites première et seconde substances thérapeutiquement actives que les doses nécessaires au traitement des mêmes affections, par l'une ou l'autre de ces mêmes dites première et seconde substances thérapeutiquement actives seules. En effet, la composition de l'invention permet d'utiliser des doses de ladite première substance thérapeutiquement active, lorsque en combinaison avec ladite seconde substance thérapeutiquement active, environ deux à cinq fois inférieures à celles nécessaires lorsque ladite première substance thérapeutiquement active est utilisée seule et des doses de ladite seconde substance thérapeutiquement active, lorsque en combinaison avec ladite première substance thérapeutiquement active, de 2 à 5 fois inférieures à celles nécessaires lorsque ladite seconde substance thérapeutiquement active est utilisée seule.

Ceci a pour conséquence d'offrir un traitement qui présente les avantages suivants:
- efficacité contre les affections sensibles avec des doses très faibles,
- efficacité contre les affections résistantes à un agent thérapeutique,
- efficacité contre les affections résistantes à plusieurs agents thérapeutiques,
- lutte contre les phénomènes de récidive,
- lutte contre les phénomènes de sélection résistance,

Dans tous ces cas, il y a une diminution remarquable des risques de toxicité et/ou d'apparition d'effets secondaires indésirables bien connus de l'homme du métier, grâce à la potentialisation qui permet l'administration de doses très faibles.

De plus, il en résulte une diminution du coût de production du traitement étant donné la faible quantité de principes actifs utilisés.

Les compositions pharmaceutiques de l'invention peuvent se présenter sous la forme de liposomes ou sous forme d'association avec des supports tels que les cyclodextrines ou les polyéthyléneglycols.

Les compositions pharmaceutiques de l'invention représentent un moyen simple et efficace pour lutter contre les problèmes liés aux affections tumorales en général qui sont essentiellement la résistance aux agents thérapeutiques et la toxicité de ceux-ci générée par l'utilisation de fortes doses.

En effet, le carvéol, le thymol, le carvacrol, et leurs mélanges sont des molécules simples n'ayant jamais été décrites comme ayant une toxicité quelconque et leur ajout ayant un effet potentialisateur sur la seconde substance thérapeutiquement active permet d'utiliser des doses beaucoup plus faibles de cette seconde substance thérapeutiquement active.

Le procédé de traitement des patients atteints d'une affection tumorale consistera donc, dans une première variante, à administrer à ces patients la dose déterminée par le médecin de la composition pharmaceutique de l'invention contenant les doses appropriées de ladite au moins une première substance thérapeutiquement active, combinées aux doses appropriées de ladite au moins une seconde substance thérapeutiquement active, c'est-à-dire l'antitumoral approprié.

Dans une seconde variante, le procédé de traitement des patients atteints d'une affection tumorale consistera à administrer à ces patients séquentiellement la dose déterminée par le médecin de ladite au moins une première substance thérapeutiquement active, puis la dose appropriée de ladite au moins une seconde substance thérapeutiquement active, c'est-à-dire l'antitumoral approprié ou l'inverse.

A cet effet, l'invention propose une trousse contenant, au moins un premier récipient contenant une desdites premières substances thérapeutiquement actives, et au moins un second récipient contenant une desdites secondes substances thérapeutiquement actives.

Cette trousse permettra au personnel soignant de préparer à la demande soit un mélange, des doses appropriées, de(s) la première(s) substance thérapeutique voulue(s) et des antitumoraux voulu(s), pour une administration simultanée, soit d'administrer séquentiellement et de façon séparée la dose appropriée de ladite au moins une première substance thérapeutiquement active, puis la dose appropriée de ladite au moins une seconde substance thérapeutiquement active, c'est-à-dire l'antitumoral approprié, ou l'inverse. Cependant, on préférera utiliser un mélange pour utilisation simultanée pour permettre au complexe de potentialisation de se former et d'agir immédiatement dès l'administration au patient.

Pour mieux faire comprendre l'invention, on va maintenant décrire à titre d'exemple un mode de mise en oeuvre.

### EXEMPLE 1 : Traitement de cellules tumorales par la doxorubicine

Des tests *in vitro* ont été menés avec des lignées cellulaires HEP (carcinome de larynx humain), BSR (carcinome rénal d'hamster) et P815 (mastocytome murin) cultivées sur milieu de culture DMEM (Dulbecco's modified eagles medium) supplémenté de 5 % de sérum foetal bovin (Gibco BRL, France), 1% de penicilline-streptomycine-neomycine, 0.2% de bicarbonate de sodium. Ces pourcentages sont des pourcentages en masse par rapport au volumes total du milieu.

Pour déterminer la concentration capable de provoquer une cytotoxicité de 50 % des cellules tumorales traitées, notée IC₅₀, d'une part de la doxorubicine (nom commercial de l'adriamycine) seule, du carvacrol seul, du thymol seul et du carvéol seul, et d'autre part, de compositions selon l'invention contenant différentes concentrations de doxorubicine en mélange avec 0,012 % (12 mg/100 ml de solution d'excipient) soit de carvacrol soit de thymol, et 0,016 % (16 mg/100 ml de solution d'excipient) de carvéol.

Dans ces compositions, l'excipient et une solution d'agar.

L'activité cytotoxique a été mesurée par la méthode MTT selon Mosmann T, 1983 (Rapid colorimetric assay for cellular growth and survival : application to prolifération and cytotoxicity assays. J Immunol Methods. 1983).

Les IC₅₀ ont été déterminées. Les résultats obtenus sont regroupés au tableau 1 qui suit dans lequel les IC₅₀ en µg/ml de chacune desdites compositions sont données pour chaque lignée cellulaire.

**Tableau 1**

| Lignée cellulaire | HEP | BSR | P815 |
|---|---|---|---|
| Carvacrol | IC₅₀ = 0, 03 | IC₅₀ = 0, 03 | IC₅₀ = 0, 03 |
| Thymol | IC₅₀ = 0, 03 | IC₅₀ = 0, 03 | IC₅₀ = 0, 03 |
| Carvéol | IC₅₀ = 0, 04 | IC₅₀ = 0, 04 | IC₅₀ = 0, 04 |
| Doxorubicine | IC₅₀ = 20 | IC₅₀ = 25 | IC₅₀ = 15 |
| Doxorubicine + 0,012 % Carvacrol | IC₅₀ = 6 | IC₅₀ = 5 | IC₅₀ = 5 |
| Doxorubicine + 0,012 % Thymol | IC₅₀ = 6 | IC₅₀ =6 | IC₅₀ =5 |
| Doxorubicine + 0,016 % Carvéol | IC₅₀ = 8 | IC₅₀ = 8 | IC₅₀ = 6 |

On voit à partir du tableau 1 que l'IC₅₀ du carvacrol seul et du thymol seul est de 0,03% (30 mg/100 ml), que celle du carvéol seul est de 0,04 % (40 mg/100 ml de solution d'excipient).
Ainsi, dans les compositions de l'invention testées, le carvacrol, le thymol et le carvéol sont à une concentration n'exerçant aucune cytotoxicité vis-à-vis des lignées cellulaires utilisées dans le test.

On voit à partir du tableau 1 que la doxorubicine à une IC₅₀, lorsqu'utilisée seule, variant entre 15 et 25 µg/ml selon la lignée cellulaire testée.

Les résultats du tableau 1 montrent clairement que la composition selon l'invention permet de diviser par trois la concentration capable de provoquer une cytotoxicité de 50 % des cellules tumorales testées de la doxorubicine.

Ainsi, les compositions selon l'invention ont une activité antitumorale remarquable en comparaison de la doxorubicine seule ou au thymol seul ou au carvacrol seul ou au carvéol seul.

De plus, une IC₅₀ de 8 µg/ml de doxorubicine n'exerce aucune cytotoxicité vis-à-vis des lignées cellulaires utilisées dans le test.

En dehors du contexte de l'invention revendiquée, des résultats similaires ont été obtenus avec d'autres familles d'agents antitumoraux connus en utilisant en tant qu'agent potentialisateur l'eugénol, le bornéol et les isomères et dérivés de ceux-ci.

Là encore, sans vouloir être liés par la théorie, les inventeurs pensent que la structure chimique des agents antitumoraux et la structure chimique des agents potentialisateurs, les agents potentialisateurs que sont le carvacrol, le thymol, le carvéol interagissent avec les agents antitumoraux pour former des complexes ayant une structure qui se diffusent facilement dans les liquides physiologiques de l'organisme et qui diffusent plus facilement dans le cytoplasme des cellules tumorales ciblées.

## Revendications

1. Composition pharmaceutique **caractérisée en ce qu'**elle comprend :
- au moins une première substance thérapeutiquement active choisie parmi le carvéol, le thymol et le carvacrol, et
- au moins une seconde substance thérapeutiquement active qui est un agent antitumoral, ledit agent antitumoral étant la doxorubicine.

2. Composition selon la revendication 1, **caractérisée en ce que** ladite première substance thérapeutiquement active est le thymol.

3. Composition selon la revendication 1, **caractérisée en ce que** ladite première substance thérapeutiquement active est le carvéol.

4. Composition selon la revendication 1, **caractérisée en ce que** ladite première substance thérapeutiquement active est le carvacrol.

5. Composition selon l'une quelconque des revendications 1 à 4, **caractérisée en ce que** lesdites première et seconde substances thérapeutiquement actives sont mises en suspension dans une solution aqueuse d'agar.

6. Composition selon l'une quelconque des revendications 1 à 5, **caractérisée en ce qu'**elle ne contient pas de détergent ou de solvant.

7. Trousse **caractérisée en ce qu'**elle contient :
- au moins un premier récipient contenant une première substance thérapeutiquement active choisie parmi le carvéol, le thymol et le carvacrol, et
- au moins un second récipient contenant une seconde substance thérapeutiquement active qui est un agent antitumoral, ledit agent antitumoral étant la doxorubicine.

8. Trousse selon la revendication 7, **caractérisée en ce que** ladite première substance thérapeutiquement active est le thymol.

9. Trousse selon la revendication 7, **caractérisée en ce que** ladite première substance thérapeutiquement active est le carvéol.

10. Trousse selon la revendication 7, **caractérisée en ce que** ladite première substance thérapeutiquement active est le carvacrol.

11. Composition selon l'une quelconque des revendications 1 à 6 ou trousse selon l'une quelconque des revendications 8 à 10 pour l'utilisation dans le traitement d'une affection due à une tumeur chez un patient.

12. Composition ou trousse pour l'utilisation selon la revendication 11, **caractérisée en ce que** les doses à administrer sont :
- entre 10 et 200 mg/kg de poids du patient/jour pour la première substance thérapeutiquement active, et
- entre 2 et 100 mg/kg de poids du patient/jour pour la seconde substance thérapeutiquement active de ladite composition.

## Claims

1. A pharmaceutical composition **characterized in that** it comprises:
- at least one first therapeutically active substance selected from the group consisting of carveol, thymol and carvacrol, and,
- at least one second therapeutically active substance which is an antitumoral agent, said antitumoral agent being doxorubicin.

2. Composition according to claim 1, **characterized in that** said first therapeutically active substance is thymol.

3. Composition according to claim 1, **characterized in that** said first therapeutically active substance is carveol.

4. Composition according to claim 1, **characterized in that** said first therapeutically active substance is carvacrol.

5. Composition according to any one of claims 1 to 4, **characterized in that** said first and second therapeutically active substances are suspended in an aqueous agar solution.

6. Composition according to any one of claims 1 to 5, **characterized in that** said composition does not include any detergent or solvent.

7. Kit **characterized in that** it comprises:
- at least one first container containing a first therapeutically active substance selected from the group consisting of carveol, thymol and carvacrol, and
- at least one second container containing a second therapeutically active substance which is an antitumoral agent, said antitumoral agent being doxorubicin.

8. Kit according to claim 7, **characterized in that** said first therapeutically active substance is thymol.

9. Kit according to claim 7, **characterized in that** said first therapeutically active substance is carveol.

10. Kit according to claim 7, **characterized in that** said first therapeutically active substance is carvacrol.

11. Composition according to any one of claims 1 to 6 or kit according to any one of claims 8 to 10 for use for the treatment of an affection due to a tumor in a patient.

12. Composition or kit for use according to claim 11, **characterized in that** doses to be administered are:
- between 10 and 200 mg/kg of body weight/day of said first therapeutically active substance, and
- between 2 and 100 mg/kg of body weight/day of said second therapeutically active substance of said composition.

## Patentansprüche

1. Pharmazeutische Zusammensetzung, **dadurch gekennzeichnet, dass** sie umfasst:
- wenigstens eine erste therapeutisch aktive Substanz, ausgewählt aus Carveol, Thymol und Carvacrol, und
- wenigstens eine zweite therapeutisch aktive Substanz, die ein Antitumormittel ist, wobei besagtes Antitumormittel Doxorubicine ist.

2. Zusammensetzung gemäß Anspruch 1, **dadurch gekennzeichnet, dass** besagte erste therapeutisch aktive Substanz Thymol ist.

3. Zusammensetzung gemäß Anspruch 1, **dadurch gekennzeichnet, dass** besagte erste therapeutisch aktive Substanz Carveol ist.

4. Zusammensetzung gemäß Anspruch 1, **dadurch gekennzeichnet, dass** besagte erste therapeutisch aktive Substanz Carvacrol ist.

5. Zusammensetzung gemäß einem der Ansprüche 1 bis 4, **dadurch gekennzeichnet, dass** besagte erste und zweite therapeutisch aktive Substanz in einer wässrigen Agarlösung suspendiert sind.

6. Zusammensetzung gemäß einem der Ansprüche 1 bis 5, **dadurch gekennzeichnet, dass** sie kein Detergens oder Lösemittel enthält.

7. Kit, **dadurch gekennzeichnet, dass** er enthält:
- wenigstens einen ersten Behälter, enthaltend eine erste therapeutisch aktive Substanz, ausgewählt aus Carveol, Thymol und Carvacrol, und
- wenigstens einen zweiten Behälter, enthaltend eine zweite therapeutisch aktive Substanz, die ein Antitumormittel ist, wobei besagtes Antitumormittel Doxorubicin ist.

8. Kit gemäß Anspruch 7, **dadurch gekennzeichnet, dass** besagte erste therapeutisch aktive Substanz Thymol ist.

9. Kit gemäß Anspruch 7, **dadurch gekennzeichnet, dass** besagte erste therapeutisch aktive Substanz Carveol ist.

10. Kit gemäß Anspruch 7, **dadurch gekennzeichnet, dass** besagte erste therapeutisch aktive Substanz Carvacrol ist.

11. Zusammensetzung gemäß einem der Ansprüche 1 bis 6 oder Kit gemäß einem der Ansprüche 8 bis 10 zur Verwendung in der Behandlung einer Tumorerkrankung bei einem Patienten.

12. Zusammensetzung oder Kit zur Verwendung gemäß Anspruch 11, **dadurch gekennzeichnet, dass** die zu verabreichenden Dosen:
- zwischen 10 und 200 mg/kg Körpergewicht des Patienten/Tag für die erste therapeutisch aktive Substanz, und
- zwischen 2 und 100 mg/kg Körpergewicht des Patienten/Tag für die zweite therapeutisch aktive Substanz besagter Zusammensetzung liegen.
